# EUROPEAN PATENT APPLICATION

(11) **EP 1 782 843 A1**
(43) Date of publication of application: **09.05.2007**
(21) Application number: 04748208.8
(22) Date of filing: 03.08.2004
(51) Int. Cl.: A61L 9/22, A61L 9/01

(54) **DEODORIZER**

(71) Applicant: MITSUBISHI DENKI K.K., Tokyo 100-8310 (JP)
(72) Inventor: FURUHASHI, Takuya, c/o Mitsubishi Denki K.K., Tokyo 100-8310 (JP); OHTA, Kohji, c/o Mitsubishi Denki K.K., Tokyo 100-8310 (JP); NAKAGAWA, Hidetomo, c/o Mitsubishi Denki K.K., Tokyo 100-8310 (JP); MORIOKA, Reiji, c/o Mitsubishi Denki K.K., Tokyo 100-8310 (JP)
(74) Representative: Pfenning, Meinig & Partner GbR
(86) International application number: PCT/JP2004/011075
(87) International publication number: WO 2006/013620

(57) **Abstract**

The present invention relates to a deodorizing apparatus that removes odorant gas components and hazardous volatile chemical substances to clean the air. The deodorizing apparatus efficiently decomposes and removes odorant gases and hazardous volatile chemical substances to clean the air with a low energy consumption. The deodorizing apparatus is placed in an air duct and includes a discharge electrode and a counter electrode to which a high voltage is applied and a catalyst unit for removing odorant gas components and hazardous volatile chemical substances. The discharge electrode is placed on the windward side of the air duct and is constituted by a flat electrode having projections. The counter electrode is placed on the leeward side of the air duct and has openings that are formed by a metal or an electrically conductive material. The catalyst unit is placed between the discharge electrode and the counter electrode, is apart from the discharge electrode at a predetermined distance, is in contact with the counter electrode, is mainly composed of manganese oxide, and has openings in the air duct of 10-1000 cells per square inch. These openings in the catalyst unit are larger than those in the counter electrode. The projections of the discharge electrode are tilted at an angle of 0-90 degrees relative to the catalyst surface of the catalyst unit.

## Description

### Technical Field

The present invention relates to a deodorizing apparatus that removes odorant gas components and hazardous volatile chemical substances to clean the air.

### Background Art

In a conventional deodorizing apparatus, a high-voltage discharge electrode and a counter electrode are placed in an air duct and a honeycomb catalyst for removing odorant gas components is placed between the discharge electrode and the counter electrode. The discharge electrode is placed on the windward side of the air duct and includes subelectrodes having a small radius of curvature. The honeycomb catalyst is made of a dielectric material, on which a catalyst or an adsorbent that adsorbs and decomposes an odorant and ozone is loaded, and has a space for the passage of the air. For example, see Patent Document 1.

Patent Document 1:
   Japanese Unexamined Patent Application Publication No. 2004-113704 (Fig. 1)

### Disclosure of Invention

### Problems to be solved by the Invention

However, the conventional deodorizing apparatus is not efficient in decomposing and removing odorant gases when using the applied electric energy.
The present invention is intended to overcome such a problem. That is, it is an object of the present invention to provide a deodorizing apparatus that can more efficiently decompose and remove odorant gases and hazardous volatile chemical substances to clean the air with a low energy consumption.

### Means for solving the problem

A deodorizing apparatus according to the present invention is placed in an air duct and includes a high-voltage discharge electrode, a counter electrode, and a catalyst unit for removing odorant gas components and hazardous volatile chemical substances. The discharge electrode is placed on the windward side of the air duct and is constituted by a flat electrode having projections. The counter electrode is placed on the leeward side of the air duct and has openings that are formed by a metal or an electrically conductive material. The catalyst unit is placed between the discharge electrode and the counter electrode, is apart from the discharge electrode at a predetermined distance, and is in contact with the counter electrode. The catalyst unit is mainly composed of manganese oxide and has openings in the air duct of 10-1000 cells per square inch. These openings are larger than the openings in the counter electrode. The projections on the discharge electrode are tilted at 0-90 degrees relative to the catalyst surface of the catalyst unit.
In the deodorizing apparatus according to the present invention, the radius of curvature at the tip of the projection on the discharge electrode is 0.0025-0.25 mm, the length of the projection is 2-5 mm, the length of the base of the projection is 0.5-1 mm, the thickness of the discharge electrode is 0.005-0.3 mm, the distance between the discharge electrode and the catalyst unit is 4-8 mm, the voltage applied between the discharge electrode and the counter electrode is 4-8 kV, and the distance between projections is 9-35 mm.
Furthermore, the deodorizing apparatus according to the present invention is placed in the air duct and includes a high-voltage discharge electrode, a counter electrode, and a catalyst unit for removing odorant gas components and hazardous volatile chemical substances. The discharge electrode is placed on the windward side of the air duct and is constituted by a flat electrode having an end thickness of 0.1 mm or less. The counter electrode is placed on the leeward side of the air duct and has openings that are formed by a metal or an electrically conductive material. The catalyst unit is placed between the discharge electrode and the counter electrode, is apart from the discharge electrode at a predetermined distance, and is in contact with the counter electrode. The catalyst unit is mainly composed of manganese oxide and has openings in the air duct of 10-1000 cells per square inch. These openings are larger than the openings in the counter electrode. The end of the discharge electrode is tilted at 0-90 degrees relative to the catalyst surface of the catalyst unit.
In the deodorizing apparatus according to the present invention, an end of the discharge electrode is cut or rolled to 0.1 mm or less in thickness.
In the deodorizing apparatus according to the present invention, an end of the discharge electrode has a radius of curvature of 0.1 mm or less.

### Effects

In a deodorizing apparatus according to the present invention, a discharge electrode is placed on the windward side of an air duct and is constituted by a flat electrode having projections. A ground electrode is placed on the leeward side of the air duct and has openings that are formed by a metal or an electrically conductive material. A catalyst filter is placed between the discharge electrode and the ground electrode, is apart from the discharge electrode at a predetermined distance, and is in contact with the ground electrode. The catalyst filter is mainly composed of manganese oxide and has openings in the air duct of 10-1000 cells per square inch. These openings are larger than the openings in the ground electrode. The projections on the discharge electrode 1 are tilted at 0-90 degrees relative to the catalyst surface of the catalyst filter. The deodorizing apparatus according to the present invention thus constructed can more efficiently decompose and remove odorant gases and hazardous volatile chemical substances to clean the air in a room with a low energy consumption.

### Brief Description of the Drawings

Fig. 1 is a perspective view of a deodorizing apparatus according to Example 1 of the present invention.
Fig. 2 is an exploded perspective view of the deodorizing apparatus according to Example 1 of the present invention.
Fig. 3 is a fragmentary enlarged perspective view of a discharge electrode 1 of the deodorizing apparatus according to Example 1 of the present invention.
Fig. 4 is a perspective view of a catalyst filter of the deodorizing apparatus according to Example 1 of the present invention.
Fig. 5 illustrates discharging between the discharge electrode and the catalyst filter of the deodorizing apparatus according to Example 1 of the present invention.
Fig. 6 shows the abilities to remove formaldehyde by discharging in the deodorizing apparatus according to Example 1 of the present invention.
Fig. 7 is a perspective view of a discharge electrode of a deodorizing apparatus according to Example 2 of the present invention.
Fig. 8 shows the abilities to remove formaldehyde for differently spaced projections on the discharge electrode of the deodorizing apparatus according to Example 2 of the present invention.
Fig. 9 shows the abilities to remove formaldehyde for different vertical distances between subelectrodes in the discharge electrode of the deodorizing apparatus according to Example 2 of the present invention.
Fig. 10 shows the abilities to remove formaldehyde for different distances between the discharge electrode and a catalyst filter of the deodorizing apparatus according to Example 2 of the present invention.
Fig. 11 is a front view of an air conditioner provided with the deodorizing apparatus according to Example 2 of the present invention without a front panel.
Fig. 12 is a sectional side view of the air conditioner provided with the deodorizing apparatus according to Example 2 of the present invention.
Fig. 13 is a perspective view of a discharge electrode of a deodorizing apparatus according to Example 3 of the present invention.
Fig. 14 is a fragmentary enlarged perspective view of the deodorizing apparatus according to Example 3 of the present invention.
Fig. 15 illustrates discharging between the discharge electrode and a catalyst filter of the deodorizing apparatus according to Example 3 of the present invention.
Fig. 16 is a perspective view of a discharge electrode of a deodorizing apparatus according to Example 4 of the present invention.
Fig. 17 is a fragmentary enlarged perspective view of the deodorizing apparatus according to Example 4 of the present invention.
Fig. 18 illustrates discharging between the discharge electrode and a catalyst filter of the deodorizing apparatus according to Example 4 of the present invention.
Fig. 19 is a perspective view of a discharge electrode of a deodorizing apparatus according to Example 5 of the present invention.
Fig. 20 is a fragmentary enlarged perspective view of the deodorizing apparatus according to Example 5 of the present invention.
Fig. 21 illustrates discharging between the discharge electrode and a catalyst filter of the deodorizing apparatus according to Example 5 of the present invention.
Fig. 22 shows a relationship between voltage and current at an end of the discharge electrode of the deodorizing apparatus according to Example 5 of the present invention.

### Reference Numerals

1: discharge electrode, 1a: projection, 1b: end, 1c: end, 1d: end, 1e: tip, 1f: end, 2: catalyst filter, 3: casing, 4: front cover, 5: ground electrode, 6: supporting cover, 7: deodorizing apparatus, 8: discharge path, 9: air conditioner, 10: heat exchanger, 11: fan.

### Best Mode for Carrying Out the Invention

### EXAMPLE 1

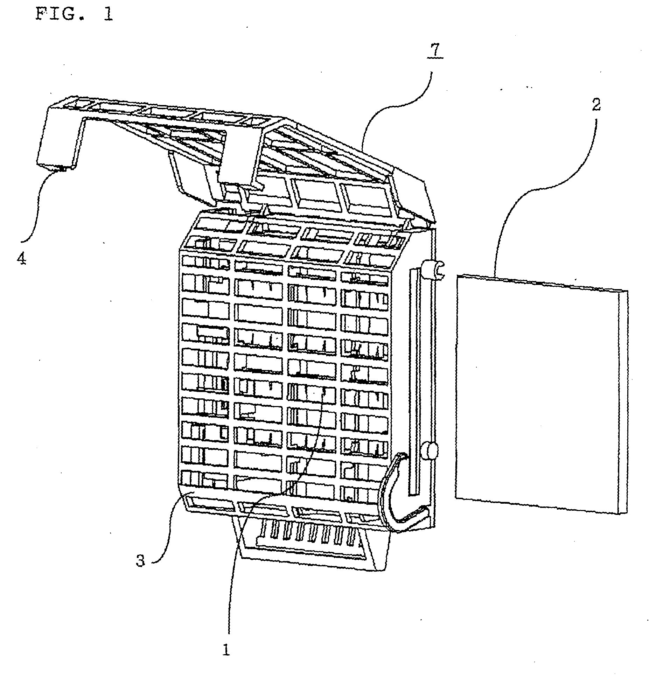

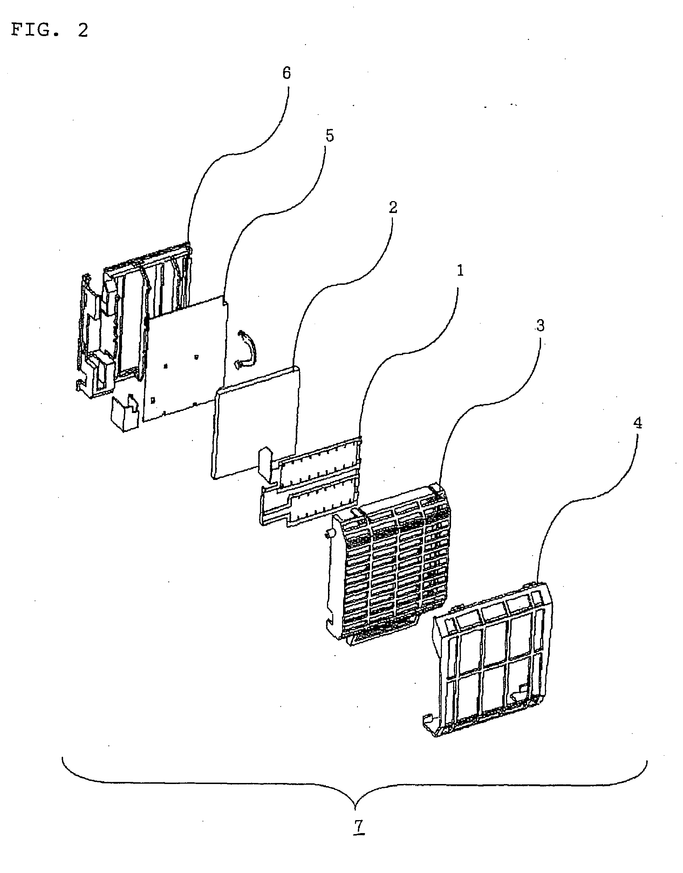

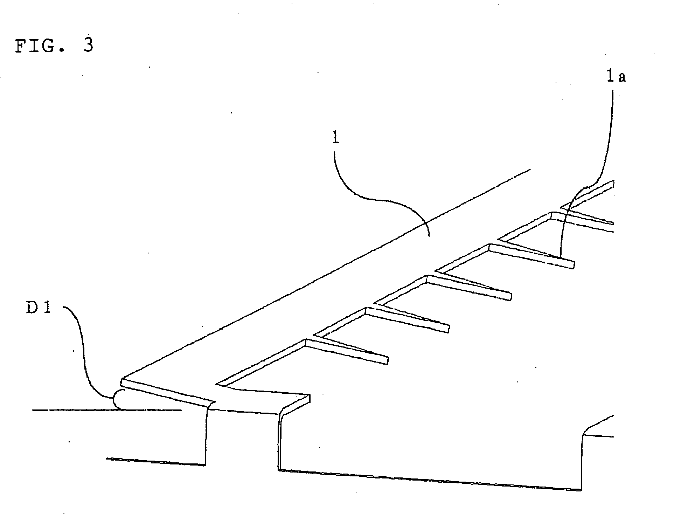

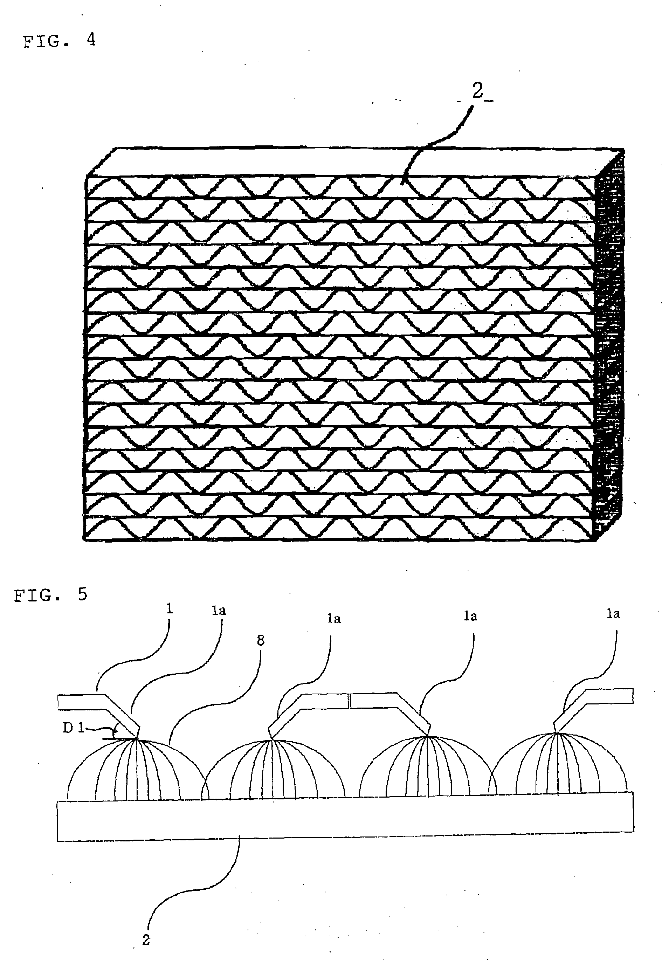

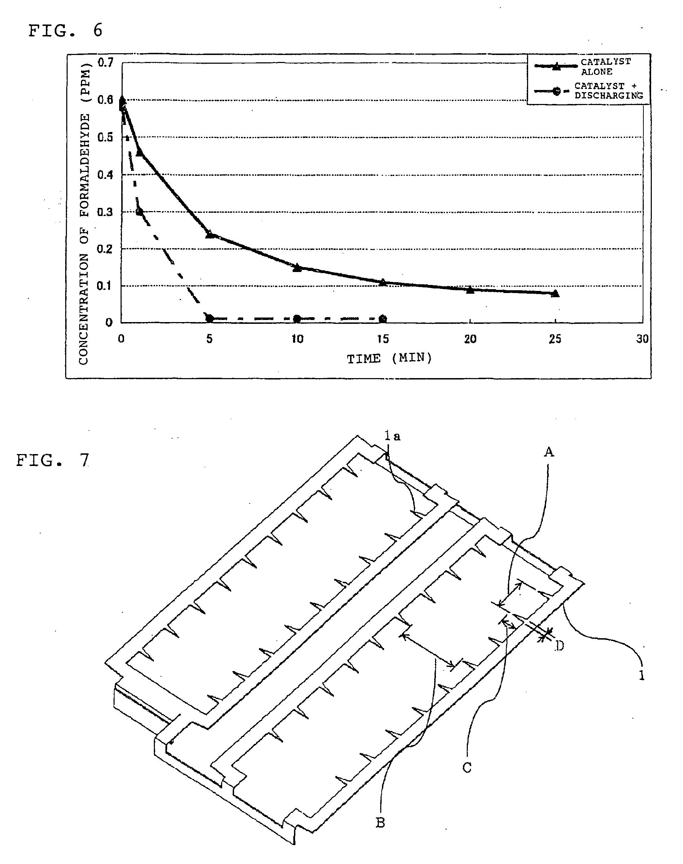

Fig. 1 is a perspective view of a deodorizing apparatus according to Example 1 of the present invention. Fig. 2 is an exploded perspective view of the deodorizing apparatus. Fig. 3 is a fragmentary enlarged perspective view showing a detailed structure of a discharge electrode of the deodorizing apparatus. Fig. 5 illustrates discharging between the discharge electrode and the catalyst filter of the deodorizing apparatus. Fig. 6 shows the abilities to remove formaldehyde by discharging in the deodorizing apparatus.
In these figures, a deodorizing apparatus 7 is made of a metal, such as stainless steel or copper, and includes a flat discharge electrode 1 having projections 1a; a catalyst filter 2 having openings, such as honeycomb or corrugated; a plastic casing 3, which covers the discharge electrode 1 and the catalyst filter 2; a front cover 4 in front of the casing 3; a ground electrode 5, which is made of an electrically conductive material, such as a metal or an electrically conductive plastic, and has openings, such as a mesh or punched holes; and a supporting cover 6 for supporting the whole deodorizing apparatus 7. The front cover 4, the casing 3, the discharge electrode 1, the catalyst filter 2, the discharge electrode 1, and the supporting cover 6 are disposed in this order from the windward side to the leeward side of an air duct. The catalyst filter 2 refers to a catalyst unit and the ground electrode 5 refers to a counter electrode. The flat discharge electrode 1 has a plurality of projections la at an end. The tips of the projections la are tilted at a certain angle D relative to the catalyst filter 2. The angle D is adjusted to 0-90 degrees.
Honeycomb or corrugated openings in the catalyst filter 2 have 10 to 1000 cells per square inch in the direction of airflow in the air duct. A dielectric substrate or a ceramic substrate of the catalyst filter 2 is impregnated with a catalyst or an adsorbent. The catalyst filter 2 mainly contains manganese oxide and also contains an oxide of iron, copper, zinc, cobalt, or nickel, and/or a noble metal, such as gold, silver, or platinum, or a mixture thereof. The catalyst filter 2 further contains an adsorbent, such as activated carbon, zeolite, or silica. The catalyst filter 2 and the discharge electrode 1 are spaced from several to several tens of millimeters apart. The catalyst filter 2 and the ground electrode 5 are in contact with each other.
The ground electrode 5 has openings formed by a metal or an electrically conductive material, such as mesh or punched holes. These openings are larger than the openings in the catalyst filter 2.
The discharge electrode 1 and the ground electrode 5 are connected to a high-voltage power supply (not shown). A direct-current voltage of several to several tens of kilovolts is applied between the discharge electrode 1 and the ground electrode 5. The catalyst filter 2, which is a composite of an insulating material and a conductive material, has some electrical conductivity and has the same electric potential as the ground electrode 5 when it is in contact with the ground electrode 5.
The operation of the deodorizing apparatus will be described below.

In such a constructed deodorizing apparatus, a direct-current voltage of several to several tens of kilovolts is applied between the discharge electrode 1 and the ground electrode 5 from the high-voltage power supply (not shown), causing corona discharge from the discharge electrode 1 to the catalyst filter 2. The larger openings in the ground electrode 5 than those in the catalyst filter 2 prevent the corona discharge from passing through the catalyst filter 2 to the ground electrode 5.
Malodorous substances and volatile organic compounds in the air, such as toluene and formaldehyde, are temporarily trapped by the catalyst filter 2 and are subsequently decomposed by active species generated by the corona discharge. Fig. 5 illustrates the corona discharge from the discharge electrode 1 to the catalyst filter 2. A voltage applied between the discharge electrode 1 and the ground electrode 5 from the high-voltage power supply causes corona discharge from the projections la of the discharge electrode 1 to the catalyst filter 2. The corona discharge forms a discharge path 8. The position of the discharge path 8 spreading over the catalyst filter 2 depends on an angle D1 of the projection 1a of the discharge electrode 1 relative to the catalyst filter 2. The decomposition is most effective when the angle D1 of the projection 1a is 90 degrees relative to the catalyst surface of the catalyst filter 2.
The corona discharge generates active species, such as an oxygen radical or a hydroxyl radical, on the catalyst filter 2. This active species decomposes and detoxifies a malodorous substance or a volatile organic compound, such as toluene or formaldehyde, improving removal efficiency. Fig. 6 shows the efficiency of removing formaldehyde with the catalyst filter 2 alone, as compared with the efficiency with the catalyst filter 2 in conjunction with the formation of the discharge path 8 by the corona discharge. Although the catalyst filter 2 alone exhibits a low removing rate, the catalyst filter 2 in conjunction with the corona discharge increases the rate of removing formaldehyde; that is, formaldehyde is almost completely removed in about 5 minutes. In addition, the catalyst filter 2 in conjunction with the corona discharge consumes 2 W of power, while the removing rate corresponds to that of a typical thermal catalyst that consumes about 400 W of heat energy. Thus, the catalyst filter 2 in conjunction with the corona discharge increases the removing rate with a very low energy consumption.

As described above, in the deodorizing apparatus 7, the discharge electrode 1 is placed on the windward side of the air duct and is constituted by a flat electrode having projections la. The ground electrode 5 is placed on the leeward side of the air duct and has openings that are formed by a metal or an electrically conductive material. The catalyst filter 2 is placed between the discharge electrode 1 and the ground electrode 5, is apart from the discharge electrode 1 at a predetermined distance, and is in contact with the ground electrode 5. The catalyst filter 2 is mainly composed of manganese oxide and has openings in the air duct of 10-1000 cells per square inch. These openings are larger than the openings in the ground electrode 5. The projections la on the discharge electrode 1 are tilted at 0-90 degrees relative to the catalyst surface of the catalyst filter 2. The deodorizing apparatus according to the present invention thus constructed can more efficiently decompose and remove odorant gases and hazardous volatile chemical substances to clean the air in a room with a low energy consumption.

### EXAMPLE 2

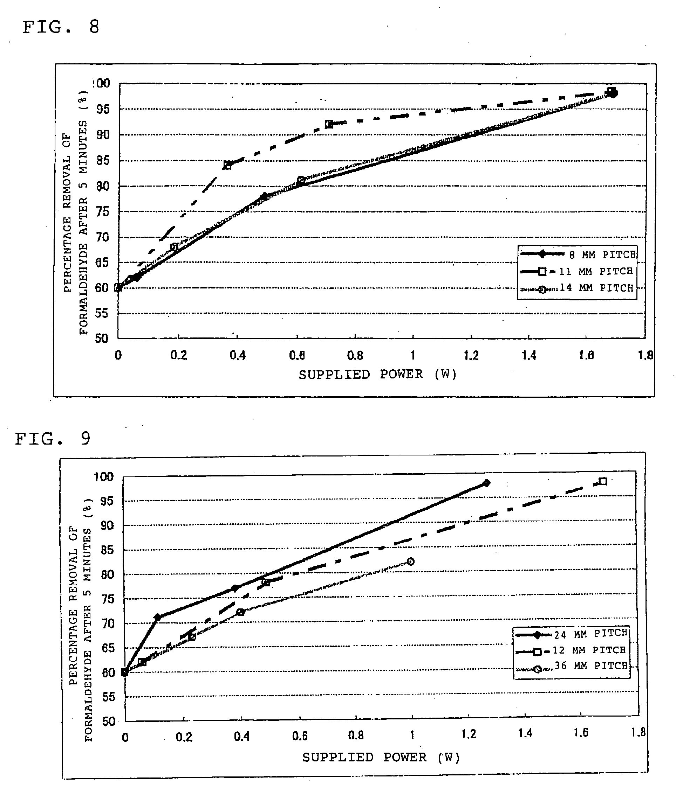

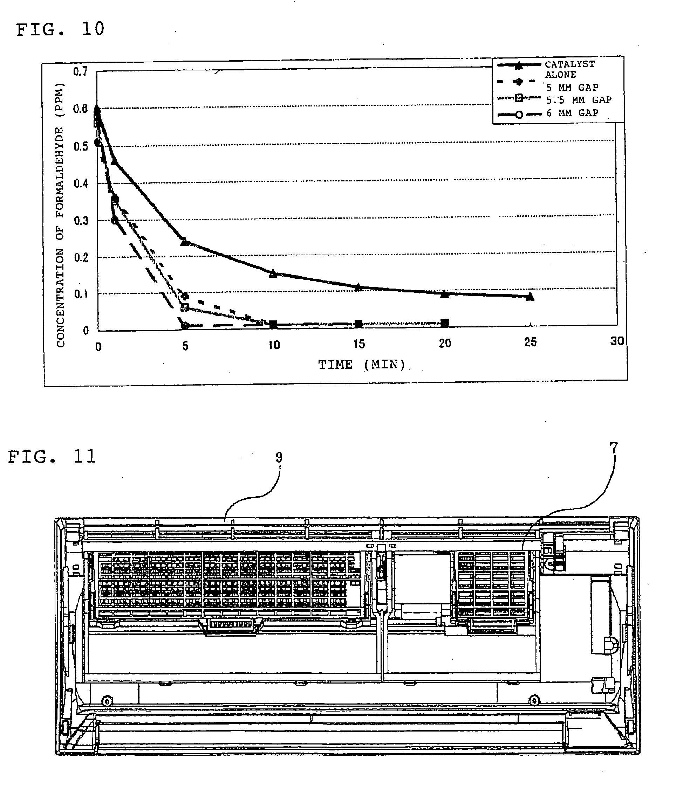

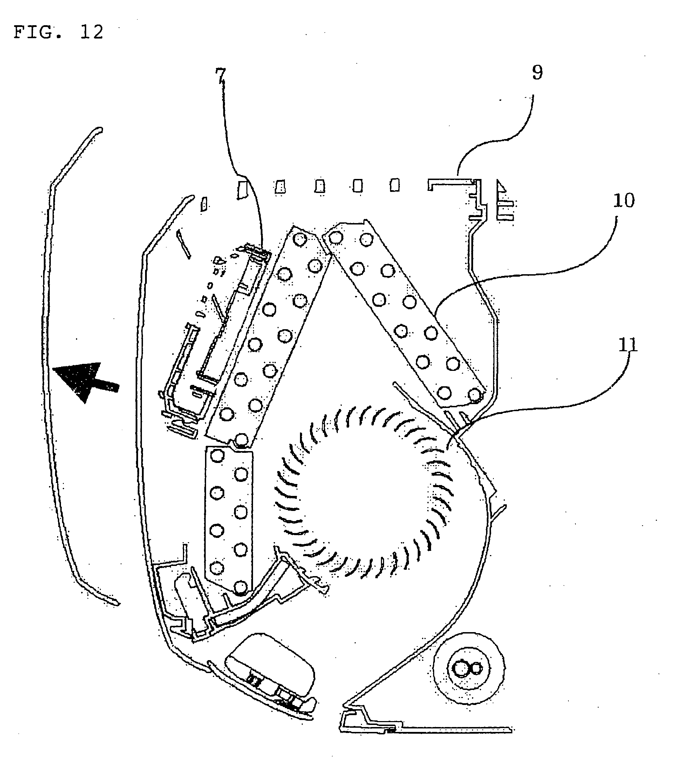

Fig. 7 is a perspective view of a discharge electrode of a deodorizing apparatus according to Example 2 of the present invention. Fig. 8 shows the abilities to remove formaldehyde for different lateral distances between adjacent projections on the discharge electrode of the deodorizing apparatus. Fig. 9 shows the abilities to remove formaldehyde for different vertical distances between opposite projections on the discharge electrode of the deodorizing apparatus. Fig. 10 shows the abilities to remove formaldehyde for different distances between the discharge electrode and a catalyst filter of the deodorizing apparatus. Fig. 11 is a front view of an air conditioner provided with the deodorizing apparatus without a front panel. Fig. 12 is a sectional side view of the air conditioner provided with the deodorizing apparatus.
Through these figures, identical or corresponding parts are denoted by like numerals as of Example 1 and will not be further explained. The lateral distance between adjacent projections 1a of the discharge electrode 1 is denoted by A, the vertical distance between opposite projections 1a is denoted by B, and the length of projection is denoted by C. The components other than the discharge electrode 1 in the deodorizing apparatus are the same as Example 1; thus, Fig. 2 can also be used here as the exploded perspective view and Fig. 5 can also be used here to illustrate discharging between the discharge electrode and the catalyst filter of the deodorizing apparatus.

The operation of the deodorizing apparatus will be described below.

In such a constructed deodorizing apparatus, as in Example 1, a direct-current voltage of several to several tens of kilovolts is applied between the discharge electrode 1 and the ground electrode 5 from the high-voltage power supply (not shown), causing corona discharge from the discharge electrode 1 to the catalyst filter 2. The larger openings in the ground electrode 5 than those in the catalyst filter 2 prevent the corona discharge from passing through the catalyst filter 2 to the ground electrode 5. Malodorous substances and volatile organic compounds in the air, such as toluene and formaldehyde, are temporarily trapped by the catalyst filter 2 and are subsequently decomposed by active species generated by the corona discharge.

Next, the ability to remove formaldehyde will be described for those projections 1a of the discharge electrode 1 that have different structures.

Fig. 8 shows the abilities to remove formaldehyde for different lateral distances A between adjacent projections as a function of supplied power. A percentage removal of formaldehyde after 5 minutes on the horizontal axis means a percentage removal of formaldehyde in a container of 1 cubic meters after 5 minutes. In Fig. 8, the projections 1a having a lateral distance (pitch) A of 11 mm exhibit higher percentage removal than the projections 1a having a lateral distance (pitch) A of 8 mm or 14 mm. This indicates that the percentage removal will not be increased when the distance A is too short or too long.
Fig. 9 shows the abilities to remove formaldehyde for different vertical distances (pitches) B between opposite projections as a function of supplied power. A percentage removal of formaldehyde after 5 minutes on the horizontal axis is the same as in Fig. 8. In Fig. 9, the projections 1a having a vertical distance (pitch) B of 34 mm exhibit higher percentage removal than the projections la having a lateral distance (pitch) B of 12 mm or 36 mm. This indicates that, as is the case with the lateral distance A, the percentage removal will not be increased when the vertical distance B is too short or too long.
Fig. 10 shows a reduction in the concentration of formaldehyde in a container of 1 cubic meters for different distances (gaps) between the tip of the projection 1a and the catalyst filter 2. The distance between the projection 1a and the catalyst filter 2 of 6 mm gives a faster reduction than the distance of 5.5 mm or 5 mm, indicating that a longer distance increases the rate of removal. The longer distance between the projection la and the catalyst filter 2 results in a wider discharge path 8, which allows active species to spread over a catalyst surface, increasing the rate of removal. Furthermore, a smaller radius of curvature at the tip of the projection la enhances discharge, improving the performance with low power consumption. Preferably, the radius of curvature at the tip of the projection la is about 0.0025-0.25 mm, the length C of the projection la is 2-5 mm, and the length D of the base of the projection la is 0.5-1 mm. The smaller thickness of the discharge electrode 1 reduces the generation of ozone and thus the thickness is preferably about 0.005-0.3 mm.
The results in Figs. 8-10 show that the distance between the discharge electrode 1 and the catalyst filter 2 is preferably 4-8 mm, an applied voltage is preferably 4-8 kV, the distance A between the projections la is preferably 9-13 mm, and the distance B between the projections la is preferably 13-35 mm.
Next, the operation of an air conditioner provided with the deodorizing apparatus will be described below.
As shown in Fig. 11, the deodorizing apparatus 7 is installed in front of a heat exchanger 10 on the upper right as one faces an air conditioner 9. In such a structure, when the air conditioner 9 is in operation and a fan 11 rotates, indoor air is taken in the deodorizing apparatus 7 and is blown into the room after cleaning.
As described above, the deodorizing apparatus 7, in which the radius of curvature at the tip of the projection la is 0.0025-0.25 mm, the length C of the projection la is 2-5 mm, the length D of the base of the projection la is 0.5-1 mm, the thickness of the discharge electrode 1 is 0.005-0.3 mm, the distance between the discharge electrode 1 and the catalyst filter 2 is 4-8 mm, the voltage applied between the discharge electrode 1 and the ground electrode 5 is 4-8 kV, and the distance B between the projections 1a is 13-35 mm, can more efficiently decompose and remove odorant gases and hazardous volatile chemical substances to clean the air in a room with a low energy consumption.

### EXAMPLE 3

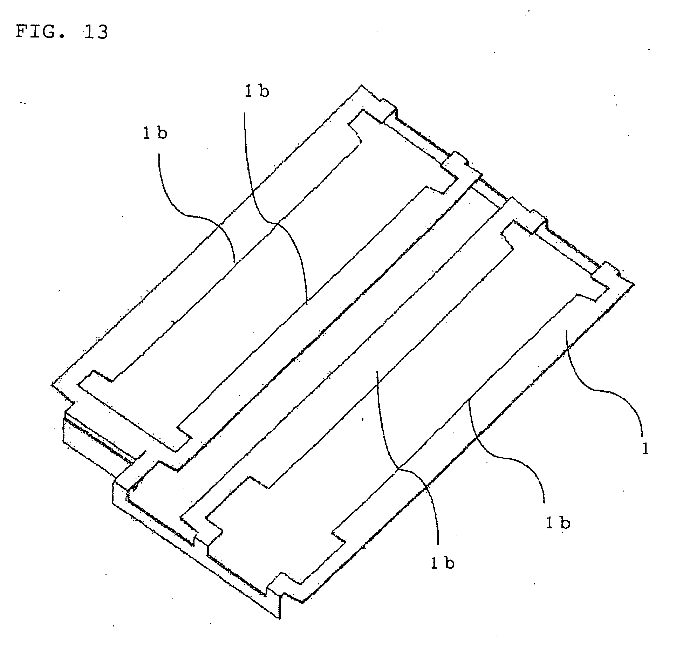

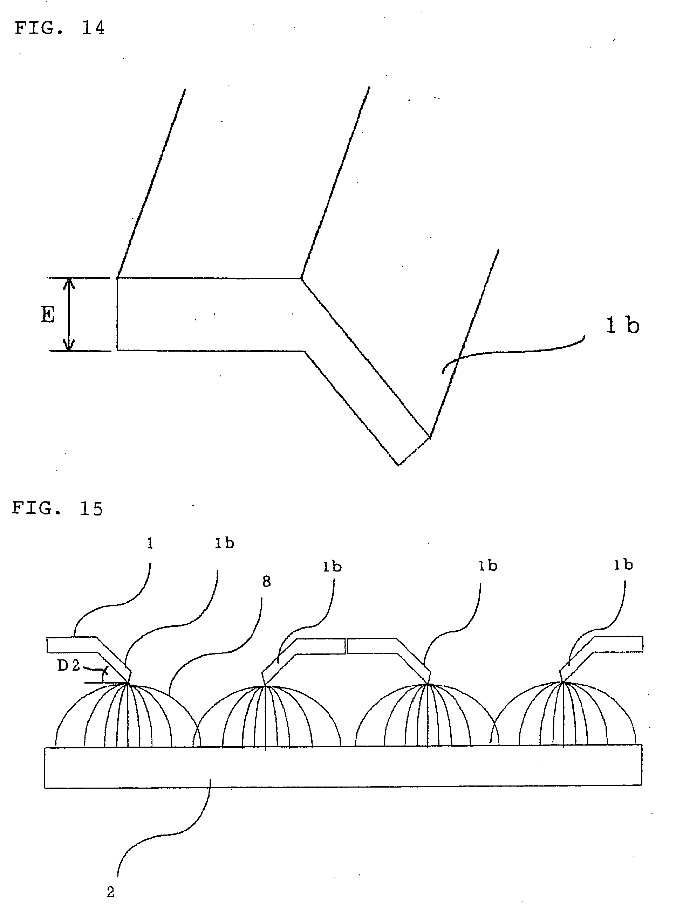

Fig. 13 is a perspective view of a discharge electrode of a deodorizing apparatus according to Example 3 of the present invention. Fig. 14 is a fragmentary enlarged perspective view of the discharge electrode of the deodorizing apparatus. Fig. 15 illustrates discharging between the discharge electrode and a catalyst filter of the deodorizing apparatus.
Through these figures, identical or corresponding parts are denoted by like numerals as of Example 1 and will not be further explained. In Example 3, a discharge electrode 1 has a flat-shaped end instead of the projections in Example 1. Structures other than the discharge electrode 1 in a deodorizing apparatus are the same as in Example 1. Thus, Fig. 2 can also be used here as the exploded perspective view.
When a direct-current voltage of several to several tens of kilovolts is applied to the discharge electrode 1, to generate a discharge to a catalyst filter 2 at a low voltage with a low energy consumption, the thickness E of the end should be 0.1 mm or less. Such a thickness E allows for discharging with a low energy consumption.

Furthermore, an end 1b of the discharge electrode 1 is tilted at a certain angle D2 relative to a catalyst filter 2. The angle D2 is adjusted to 0-90 degrees.

The operation of the deodorizing apparatus will be described below.

In such a constructed deodorizing apparatus, a direct-current voltage of several to several tens of kilovolts is applied between the discharge electrode 1 and the ground electrode 5 from the high-voltage power supply (not shown), causing corona discharge from the end 1b of the discharge electrode 1 to the catalyst filter 2. The larger openings in the ground electrode 5 than those in the catalyst filter 2 prevent the corona discharge from passing through the catalyst filter 2 to the ground electrode 5.
Malodorous substances and volatile organic compounds in the air, such as toluene and formaldehyde, are temporarily trapped by the catalyst filter 2 and are subsequently decomposed by active species generated by the corona discharge. Fig. 15 illustrates the corona discharge from the discharge electrode 1 to the catalyst filter 2. A voltage applied between the discharge electrode 1 and the ground electrode 5 from the high-voltage power supply causes corona discharge from the end 1b of the discharge electrode 1 to the catalyst filter 2. The corona discharge forms a discharge path 8. The position of the discharge path 8 spreading over the catalyst filter 2 depends on an angle D2 of the end 1b of the discharge electrode 1 relative to the catalyst filter 2. The decomposition is most effective when the angle D2 of the end 1b is 90 degrees relative to the catalyst surface of the catalyst filter 2.
The corona discharge generates active species, such as an oxygen radical or a hydroxyl radical, on the catalyst filter 2. This active species decomposes and detoxifies a malodorous substance or a volatile organic compound, such as toluene or formaldehyde, improving removal efficiency. In addition, the catalyst filter 2 in conjunction with the corona discharge consumes 2 W of power, while the removing rate corresponds to that of a typical thermal catalyst that consumes 400 W of heat energy. Thus, the catalyst filter 2 in conjunction with the corona discharge can increase the removing rate with a very low energy consumption.
As described above, in the deodorizing apparatus 7, the discharge electrode 1 is placed on the windward side of the air duct and is constituted by a flat electrode having an end 1b 0.1 mm or less in thickness. The ground electrode 5 is placed on the leeward side of the air duct and has openings that are formed by a metal or an electrically conductive material. The catalyst filter 2 is placed between the discharge electrode 1 and the ground electrode 5, is apart from the discharge electrode 1 at a predetermined distance, and is in contact with the ground electrode 5. The catalyst filter 2 is mainly composed of manganese oxide and has openings in the air duct of 10-1000 cells per square inch. These openings are larger than the openings in the ground electrode 5. The end 1b of the discharge electrode 1 is tilted at 0-90 degrees relative to the catalyst surface of the catalyst filter 2. The deodorizing apparatus thus constructed can more efficiently decompose and remove odorant gases and hazardous volatile chemical substances to clean the air in a room with a low energy consumption.

### EXAMPLE 4

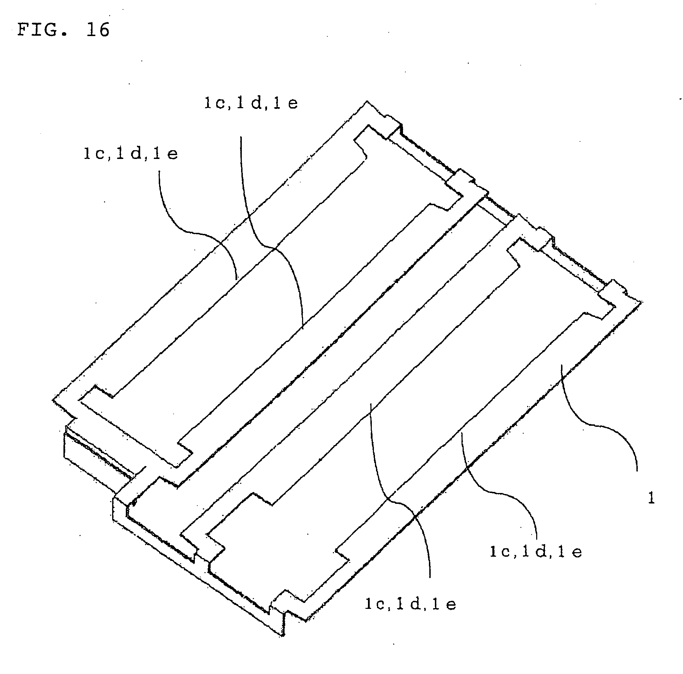

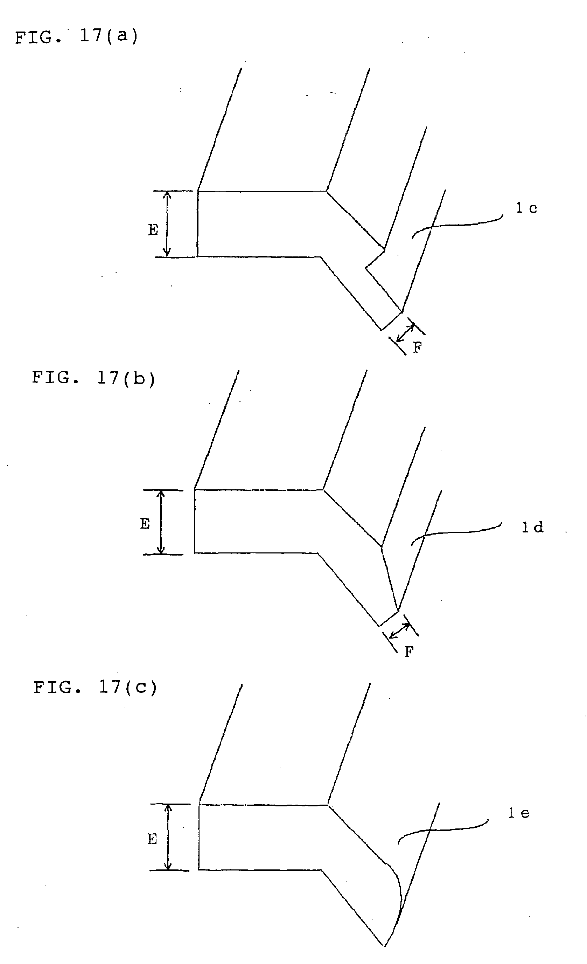

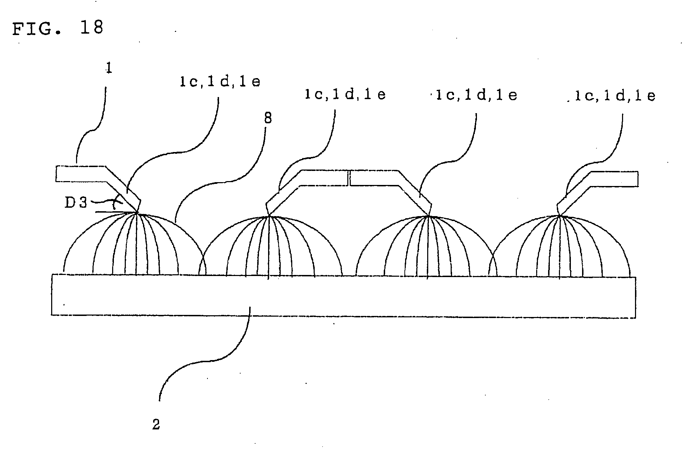

Fig. 16 is a perspective view of a discharge electrode of a deodorizing apparatus according to Example 4 of the present invention. Fig. 17 is a fragmentary enlarged perspective view of the discharge electrode of the deodorizing apparatus. Fig. 18 illustrates discharging between the discharge electrode and a catalyst filter of the deodorizing apparatus.
Through these figures, identical or corresponding parts are denoted by like numerals as of Examples 1 and 3 and will not be further explained. In Example 4, a discharge electrode 1 has one of three types of flat-shaped ends shown in Figs. 17(a), (b), and (c). In Fig. 17(a), an end 1c of the discharge electrode 1 that faces a catalyst filter 2 is rolled to have a thickness F, which is smaller than the thickness E of the electrode and is 0.1 mm or less. In Fig. 17(b), an end 1d of the discharge electrode 1 that faces the catalyst filter 2 is rolled into a different shape from the end 1c, that is, into a beveled shape. The thickness F of the end 1d is smaller than the thickness E of the discharge electrode 1 and is 0.1 mm or less. In Fig. 17(c), a tip 1e of the discharge electrode 1 that faces the catalyst filter 2 is rolled into a different shape from the ends 1c and 1d, that is, into a shape having a curved top surface. The tip has a smaller thickness than the thickness E of the discharge electrode 1 and has a thickness of 0.1 mm or less. These shapes in Figs. 17(a) to (c) increase the electric field intensity at an end of the discharge electrode 1 and thereby enhance discharging.
The ends 1c and 1d and the tip 1e of the discharge electrode 1 are tilted at a certain angle D3 relative to the catalyst filter 2. The angle D3 is adjusted to 0-90 degrees. A structure other than the discharge electrode 1 in the deodorizing apparatus is the same as in Examples 1 and 3. Thus, Fig. 2 can also be used here as the exploded perspective view.
The operation of the deodorizing apparatus will be described below.

In such a constructed deodorizing apparatus, a direct-current voltage of several to several tens of kilovolts is applied between the discharge electrode 1 and the ground electrode 5 from the high-voltage power supply (not shown), causing corona discharge from the ends 1c and 1d and the tip 1e of the discharge electrode 1 to the catalyst filter 2. The larger openings in the ground electrode 5 than those in the catalyst filter 2 prevent the corona discharge from passing through the catalyst filter 2 to the ground electrode 5.
Malodorous substances and volatile organic compounds in the air, such as toluene and formaldehyde, are temporarily trapped by the catalyst filter 2 and are subsequently decomposed by active species generated by the corona discharge. Fig. 18 illustrates the corona discharge from the discharge electrode 1 to the catalyst filter 2. A voltage applied between the discharge electrode 1 and the ground electrode 5 from the high-voltage power supply causes corona discharge from the end 1c, 1d, or the tip 1e of the discharge electrode 1 to the catalyst filter 2. The corona discharge forms a discharge path 8. The position of the discharge path 8 spreading over the catalyst filter 2 depends on an angle D3 of the end 1c, 1d, or the tip 1e of the discharge electrode 1 relative to the catalyst filter 2. The decomposition is most effective when the angle D3 of the end 1c, 1d, or the tip 1e is 90 degrees relative to the catalyst surface of the catalyst filter 2.
The corona discharge generates active species, such as an oxygen radical or a hydroxyl radical, on the catalyst filter 2. This active species decomposes and detoxifies a malodorous substance or a volatile organic compound, such as toluene or formaldehyde, improving removal efficiency. In addition, the catalyst filter 2 in conjunction with the corona discharge consumes 2 W of power, while the removing rate corresponds to that of a typical thermal catalyst that consumes 400 W of heat energy. Thus, the catalyst filter 2 in conjunction with the corona discharge can increase the removing rate with a very low energy consumption.

As described above, the deodorizing apparatus 7 is cut or rolled to have a thickness of 0.1 mm or less at the ends 1c and 1d and the tip 1e and can thereby more efficiently decompose and remove odorant gases and hazardous volatile chemical substances to clean the air in a room with a low energy consumption.

### EXAMPLE 5

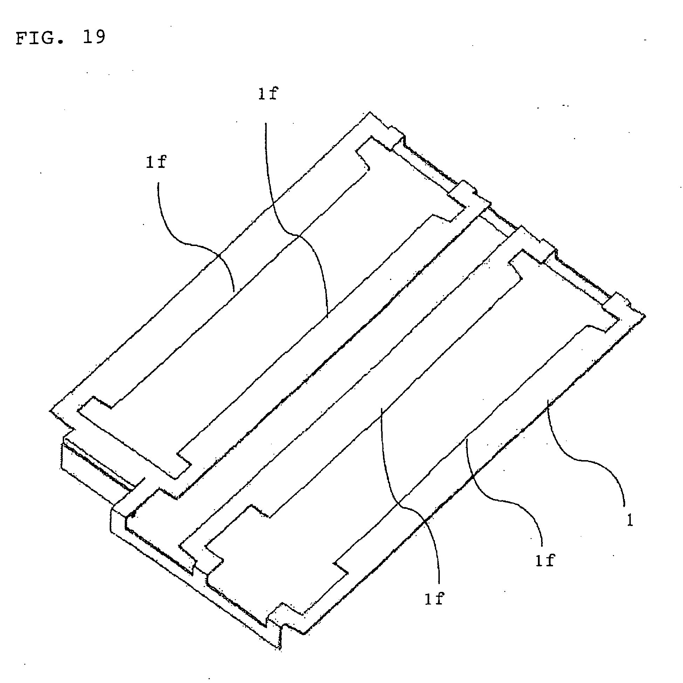

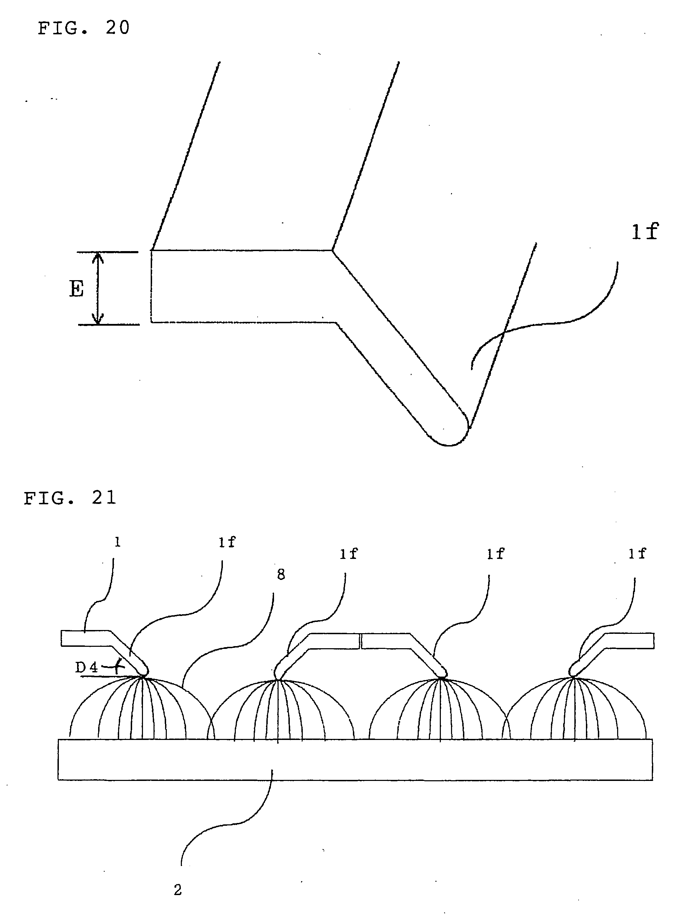

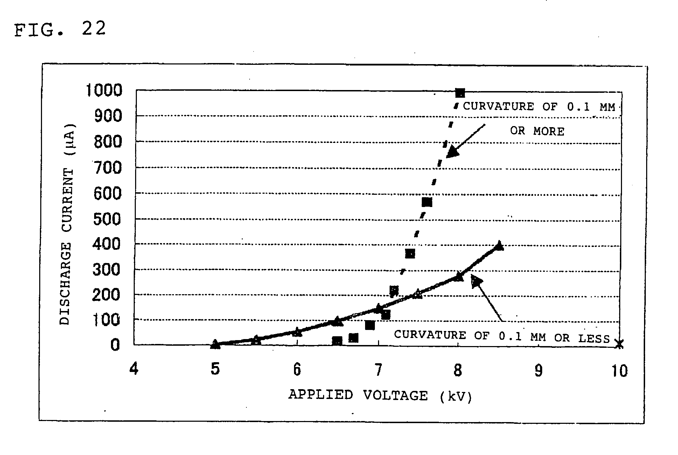

Fig. 19 is a perspective view of a discharge electrode of a deodorizing apparatus according to Example 5 of the present invention. Fig. 20 is a fragmentary enlarged perspective view of the discharge electrode of the deodorizing apparatus. Fig. 21 illustrates discharging between the discharge electrode and a catalyst filter of the deodorizing apparatus. Fig. 22 shows relationships between voltage and current for curvatures of 0.1 mm or more and 0.1 mm or less at an end of the discharge electrode of the deodorizing apparatus.
Through these figures, identical or corresponding parts are denoted by like numerals as of Examples 1 and 3 and will not be further explained. In this Example 5, the discharge electrode 1 has a flat shape and the radius of curvature of an end 1f is 0.1 mm or less. Such a shape enhances discharging and prevents a rapid increase in electric current, improving the controllability of electric current. As shown in Fig. 22, the break-down voltage for the radius of curvature of 0.1 mm or less is about 5 kV, which is lower than about 6.5 kV for the radius of curvature of 0.1 mm or more. This indicates that no rapid increase in electric current occurs.

The end 1f of the discharge electrode 1 is tilted at a certain angle D4 relative to the catalyst filter 2. The angle D4 is adjusted to 0-90 degrees.
A structure other than the discharge electrode 1 in the deodorizing apparatus is the same as in Examples 1 and 3. Thus, Fig. 2 can also be used here as the exploded perspective view.

The operation of the deodorizing apparatus will be described below.

In such a constructed deodorizing apparatus, a direct-current voltage of several to several tens of kilovolts is applied between the discharge electrode 1 and the ground electrode 5 from the high-voltage power supply (not shown), causing corona discharge from the end 1f of the discharge electrode 1 to the catalyst filter 2. The larger openings in the ground electrode 5 than those in the catalyst filter 2 prevent the corona discharge from passing through the catalyst filter 2 to the ground electrode 5.
Malodorous substances and volatile organic compounds in the air, such as toluene and formaldehyde, are temporarily trapped by the catalyst filter 2 and are subsequently decomposed by active species generated by the corona discharge. Fig. 21 illustrates the corona discharge from the discharge electrode 1 to the catalyst filter 2. A voltage applied between the discharge electrode 1 and the ground electrode 5 from the high-voltage power supply causes corona discharge from the end 1f of the discharge electrode 1 to the catalyst filter 2. The corona discharge forms a discharge path 8. The position of the discharge path 8 spreading over the catalyst filter 2 depends on an angle D4 of the end 1f of the discharge electrode 1 relative to the catalyst filter 2. The decomposition is most effective when the angle D4 of the end 1f is 90 degrees relative to the catalyst surface of the catalyst filter 2.
The corona discharge generates active species, such as an oxygen radical or a hydroxyl radical, on the catalyst filter 2. This active species decomposes and detoxifies a malodorous substance or a volatile organic compound, such as toluene or formaldehyde, improving removal efficiency. In addition, the catalyst filter 2 in conjunction with the corona discharge consumes 2 W of power, while the removing rate corresponds to that of a typical thermal catalyst that consumes 400 W of heat energy. Thus, the catalyst filter 2 in conjunction with the corona discharge can increase the removing rate with a very low energy consumption.

As described above, the deodorizing apparatus 7 has a radius of curvature of 0.1 mm or less at the end 1f and can thereby prevent a rapid increase in discharge current and has an improved controllability of electric current. In addition, the deodorizing apparatus 7 can more efficiently decompose and remove odorant gases and hazardous volatile chemical substances to clean the air in a room with a low energy consumption.

### Industrial Applicability

A deodorizing apparatus according to the present invention is suitable for cleaning indoor air.

## Claims

1. A deodorizing apparatus in an air duct, comprising:
a discharge electrode and a counter electrode to which a high voltage is applied; and
a catalyst unit for removing odorant gas components and hazardous volatile chemical substances,
wherein the discharge electrode is placed on the windward side of the air duct and is constituted by a flat electrode having projections,
the counter electrode is placed on the leeward side of the air duct and has openings that are formed by a metal or an electrically conductive material,
the catalyst unit is placed between the discharge electrode and the counter electrode, is apart from the discharge electrode at a predetermined distance, is in contact with the counter electrode, is mainly composed of manganese oxide, and has openings in the air duct of 10-1000 cells per square inch, the openings in the catalyst unit being larger than the openings in the counter electrode, and
the projections on the discharge electrode are tilted at 0-90 degrees relative to the catalyst surface of the catalyst unit.

2. The deodorizing apparatus according to Claim 1, wherein the radius of curvature at an end of the projection on the discharge electrode is 0.0025-0.25 mm, the length of projection is 2-5 mm, the length of the base of projection is 0.5-1 mm, the thickness of the discharge electrode is 0.005-0.3 mm, the distance between the discharge electrode and the catalyst unit is 4-8 mm, a voltage applied between the discharge electrode and the counter electrode is 4-8 kV, and the distance between the projections is 9-35 mm.

3. A deodorizing apparatus in an air duct, comprising:
a discharge electrode and a counter electrode to which a high voltage is applied; and
a catalyst unit for removing odorant gas components and hazardous volatile chemical substances,
wherein the discharge electrode is placed on the windward side of the air duct and is constituted by a flat electrode having an end thickness of 0.1 mm or less,
the counter electrode is placed on the leeward side of the air duct and has openings that are formed by a metal or an electrically conductive material,
the catalyst unit is placed between the discharge electrode and the counter electrode, is apart from the discharge electrode at a predetermined distance, is in contact with the counter electrode, is mainly composed of manganese oxide, and has openings in the air duct of 10-1000 cells per square inch, the openings in the catalyst unit being larger than the openings in the counter electrode, and
the ends on the discharge electrode are tilted at 0-90 degrees relative to the catalyst surface of the catalyst unit.

4. The deodorizing apparatus according to Claim 3, wherein the end of the discharge electrode is cut or rolled to have a thickness of 0.1 mm or less.

5. The deodorizing apparatus according to Claim 3 or 4, wherein the end of the discharge electrode has a radius of curvature of 0.1 mm or less.
